Europäisches Patentamt

European Patent Office

Office européen des brevets

(19)

(11) Veröffentlichungsnummer: **0 100 890**
**B1**

(12) **EUROPÄISCHE PATENTSCHRIFT**

(45) Veröffentlichungstag der Patentschrift:
**08.10.86**

(51) Int. Cl.⁴: **C 08 F 220/18, A 61 K 7/11**

(21) Anmeldenummer: **83106777.2**

(22) Anmeldetag: **11.07.83**

(54) **Copolymerisate und ihre Verwendung in Haarbehandlungsmitteln.**

(30) Priorität: **22.07.82 DE 3227334**

(43) Veröffentlichungstag der Anmeldung:
**22.02.84 Patentblatt 84/8**

(45) Bekanntmachung des Hinweises auf die Patenterteilung:
**08.10.86 Patentblatt 86/41**

(84) Benannte Vertragsstaaten:
**AT BE CH DE FR GB IT LI NL SE**

(56) Entgegenhaltungen:
**DE - A - 2 817 369**
**FR - A - 2 393 011**
**FR - A - 2 424 738**
**GB - A - 889 291**

(73) Patentinhaber: **BASF Aktiengesellschaft,
Carl-Bosch-Strasse 38, D-6700 Ludwigshafen (DE)**

(72) Erfinder: **Straub, Ferdinand, Dr., Ziegelstrasse 20,
D-6832 Hockenheim (DE)**
Erfinder: **Sanner, Axel, Dr., Lorscher Ring 2C,
D-6710 Frankenthal (DE)**
Erfinder: **Seib, Karl, Dr., Kriemhildstrasse 36,
D-6940 Weinheim (DE)**
Erfinder: **Linke, Wolfgang, Dr., An der Froschlache 7,
D-6700 Ludwigshafen (DE)**

## Beschreibung

Die vorliegende Erfindung betrifft Copolymerisate, ihre Verwendung und ein Haarbehandlungsmittel, das universell als Filmbildner in Haarsprays und Haarfestigern sowie als Hilfsmittel in Shampoos und Haarkuren einsetzbar ist.

Für die Haarkosmetik werden wegen ihrer vorteilhaften Eigenschaften häufig Polymere mit sauren und/oder basischen Gruppen eingesetzt. Die bisherigen Hilfsmittel mit ionischen Gruppen sind zwar in neutralisierter Form gut wasserlöslich, meist aber schlecht verträglich mit unpolaren Treibgasen auf Alkanbasis, die aus ökologischen Gründen in zunehmendem Masse als Ersatz für Fluorkohlenwasserstoffe Verwendung finden. Eine Möglichkeit, die Verträglichkeit der Polymeren mit den unpolaren Treibgasen zu verbessern, ist in der USP 4 192 861 beschrieben. Darin wird vorgeschlagen, zur Verbesserung der Löslichkeit die Neutralisation der Filmbildner mit langkettigen Aminen durchzuführen. Die Verwendung von langkettigen Aminen bringt aber Geruchsprobleme mit sich, und ausserdem sind solche Amine aus toxikologischen Gründen nicht erwünscht. Mit dem bisher üblichen 2-Amino-2-methylpropanol als Neutralisierungsmittel ist es zwar möglich, wasserhaltige Haarsprayformulierungen herzustellen (USP 4 261 972); die wasserhaltigen Haarsprays führen aber zu nassen Haaren, was nicht erwünscht ist. Andere Formulierungen enthalten verschiedene Wirkstoffe und Hilfsmittel im Gemisch (siehe DE-OS 30 44 738) und werden meist in wässriger Lösung als Shampoos oder zur Nachbehandlung gewaschener Haare verwendet. Es ist bekannt, dass Filmbildner mit ionischen Gruppen eine bessere Adhäsion auf den Haaren haben und besondere Wirkungen zeigen; ihre Formulierung in anderen Lösungsmitteln als Wasser und Alkoholen ist aber schwierig.

In der FR-A-2 393 011 werden durch radikalische Polymerisation erhaltene Copolymerisate aus einem eine quartäre Ammoniumgruppe enthaltenden Alkylester der (Meth)Acrylsäure, (Meth)Acrylsäure als carboxylgruppenhaltigem Monomeren, (Meth)Acrylamid und beispielsweise einem Methyl- oder Ethyl(meth)acrylat beschrieben. Als amphotere Polyelektrolyte werden diese Copolymerisate insbesondere als Papierzusatzmittel, Entwässerungsmittel oder Flockungsmittel verwendet. Sie werden auch als Komponenten für Kosmetika, wie Haarsprays und Lotionen, vorgeschlagen. Nachteilig an einer praktischen Verwendung für Haarsprays ist, dass sie aufgrund ihres ionischen Charakters bevorzugt in Wasser und kaum in den organischen Lösungsmitteln, wie sie bei Haarsprays verwendet werden, löslich sind.

Das Ziel der vorliegenden Erfindung besteht somit in der Bereitstellung eines Filmbildners, der sich durch hervorragende haarfestigende Eigenschaften auszeichnet, und der mit polaren Lösungsmitteln, wie Wasser oder Alkoholen, und mit unpolaren Treibgasen, wie Propan und Butan, gleichermassen verträglich ist, bzw. sich in den genannten Flüssigkeiten gut lösen lässt.

Die Lösung der gestellten Aufgabe wird erreicht durch: Copolymerisate, erhalten durch radikalische Copolymerisation von einem Alkylester der (Meth)acrylsäure, einem stickstoffhaltigen, neutral reagierenden wasserlöslichen Monomeren, einem kationische Gruppen enthaltenden Monomeren und einer olefinisch ungesättigten $C_3$- bis $C_4$-Carbonsäure, dadurch gekennzeichnet, dass sie durch radikalische Copolymerisation von

a) 20 bis 75 Gew.-% Ethyl-, n- oder tert.-Butyl- oder Lauryl(meth)acrylat oder deren Gemischen,

b) 5 bis 50 Gew.% N-Vinylpyrrolidon, N-Vinylcaprolactam oder deren Gemischen,

c) 1 bis 25 Gew.-% N-Vinylimidazol, 1-Vinyl-2-methyl-imidazol oder deren Gemischen und

d) 1 bis 25 Gew.-% (Meth)acrylsäure erhalten werden und einen K-Wert nach Fikentscher von 15 bis 75, gemessen in Ethanol bei 25 °C, aufweisen.

Als wasserunlösliche Monomere (a) werden Ethylacrylat, Ethylmethacrylat, n- und tertiär-Butylacrylat bzw. -methacrylat oder Lauryl(meth)-acrylat eingesetzt. Auch Gemische dieser Acryl- bzw. Methacrylester können verwendet werden.

Wasserlösliche, stickstoffhaltige und neutral reagierende Monomere (b) sind N-Vinylpyrrolidon oder N-Vinylcaprolactam oder ihre Mischungen zu nennen.

Diese N-Vinyllactame zeichnen sich durch ihre gute Löslichkeit in Wasser und in organischen Lösungsmitteln, wie Alkoholen, aus, und diese Eigenschaften übertragen sich auf die Copolymeren, obwohl diese N-Vinyllactame nur zu 5–50 Gew.-% eingesetzt werden; bevorzugt sind Mengen von 10–40 Gew.-%.

Je nach Bedarf werden die Monomeren (c) mit kationischen Gruppen in Mengen von 1–25 Gew.-% eingesetzt; man kann in Relation zu den anionischen Monomeren (d) stöchiometrische Mengen an kationischen Monomeren einsetzen und erhält dabei ein sogenanntes «inneres» Salz. Es ist aber auch möglich, einen Unterschuss oder einen Überschuss an basischen Monomeren zu verwenden.

Als Monomere mit basischen Gruppen werden die radikalisch polymerisierbaren ungesättigten Basen N-Vinylimidazol und 1-Vinyl-2-methylimidazol oder ihre Mischungen verwendet.

Als Monomere d) mit anionischen Gruppen werden in Mengen von 1–25 Gew.%, bevorzugt 5–15 Gew.-%, Acrylsäure und Methacrylsäure eingesetzt.

Die erfindungsgemässen Copolymeren können nach den üblichen Polymerisationsverfahren der Lösungs-, Fällungs-, oder Suspensionspolymerisation hergestellt werden. Bevorzugt ist ein Verfahren, bei dem die Monomeren langsam zu einem polymerisierenden Gemisch zudosiert werden, da so eine gleichmässige Polymerisation gewährleistet wird. Als Initiatoren für die Polymerisation werden die üblichen Peroxide, wie Benzoylperoxid, tert.-Butylperpivalat, tert.-Butylper-2-ethylhexanoat, Di-tert-butylperoxid, tert-Butylhydroperoxid sowie Azostarter wie Azo-bisisobutyronitril in Mengen von 0,5–5 Gew.-% verwendet. Die Molgewichte der Polymeren liegen zwischen 5000–300 000, was etwa einen K-Wert von 15–75 entspricht. Die K-Werte wurden gemäss Fikentscher «Cellulosechemie» 13, 58–64 (1932) bei 25 °C in Ethanol als Lösungsmittel bestimmt. Die nun folgenden Beispiele erläutern die Erfindung.

Beispiele
A. Herstellung des Filmbildners
Beispiel 1
In einen 1 l Kolben mit Rührer, Rückflusskühler und zwei Zulauftropftrichtern werden 10% einer Mischung aus 90 Gew.-Teilen Vinylpyrrolidon, 160 Gew.-Teilen tert-Butylacrylat, 26 Gew.-Teilen Methacrylsäure, 28 Gew.-Teilen Vinylimidazol und 300 Gew.-Teilen Isopropanol sowie 10% einer Mischung aus 50 Gew.-Teilen Isopropanol und 45 Gew.-Teilen tert-Butylperpivalat vorgelegt, der Inhalt bis zum Sieden hochgeheizt und unter Rühren die restlichen 90% der Mischungen innerhalb von 7 Stunden gleichmässig zugefahren. Es entsteht eine klare, gelbliche, viskose Lösung. Nach Beendigung des Zufahrens wird noch zwei Stunden bei Temperatur belassen und dann abgekühlt. Zu einer 3%igen isopropanolischen Lösung können bei 0 °C 69% Propan/Butan zugegeben werden, bis eine Trübung auftritt.

Nach den Angaben gemäss Beispiel 1 wurden folgende Polymere hergestellt.

| Bei-spiel | ungesättigte Säure | kationisches Monomere | wasser-lösliches Monomere | wasser-unlösliches Monomere | Löslichkeit | |
|---|---|---|---|---|---|---|
| | | | | | Alkohol | Propan/Butan |
| 2 | 54 MAS | 54 VI | 60 NVP | 132 tBA | klar | 46% |
| 3 | 13,5 MAS | 13,5 VI | 60 NVP | 213 tBA | klar | 76% |
| 4 | 27 MAS | 27 VI | 60 NVP | 186 EA | klar | 35% |
| 5 | 21 AS | 27 VI | 60 NVP | 192 tBA | klar | 65% |
| 6 | 27 MAS | 27 VI | 60 NVP | 186 LA | klar | 92% |

MAS = Methacrylsäure, AS = Acrylsäure, VI = Vinylimidazol
NVP = N-Vinylpyrrolidon, EA = Ethylacrylat, LA = Laurylacrylat,
tBA = tert. Butylacrylat

B) Haarsprayformulierungen

7. Copolymerisat gemäss Beispiel 1　　4,0 Gew.-%
Ethanol/Isopropanol　　66,0 Gew.-%
Propan/Butan 40:60　　30,0 Gew.-%
Trübungspunkt　　−35 °C

8. Copolymerisat gemäss Beispiel 1　　4,0 Gew.-%
Ethanol　　56,0 Gew.-%
Methylenchlorid　　10,0 Gew.-%
Propan/Butan 40:60　　30,0 Gew.-%
Trübungspunkt　　−35 °C

9. Copolymerisat gemäss Beispiel 1　　4,0 Gew.-%
Ethanol　　31,0 Gew.-%
Methylenchlorid　　35,0 Gew.-%
Propan/Butan 40:60　　30,0 Gew.-%
Trübungspunkt　　−35 °C

**Patentansprüche**

1. Copolymerisate, erhalten durch radikalische Copolymerisation von einem Alkylester der (Meth)acrylsäure, einem stickstoffhaltigen, neutral reagierenden wasserlöslichen Monomeren, einem kationische Gruppen enthaltenden Monomeren und einer olefinisch ungesättigten $C_3$- bis $C_4$-Carbonsäure, dadurch gekennzeichnet, dass sie durch radikalische Copolymerisation von
a) 20 bis 75 Gew.-% Ethyl-, n- oder tert.-Butyl- oder Lauryl(meth)acrylat oder deren Gemischen,
b) 5 bis 50 Gew.-% N-Vinylpyrrolidon, N-Vinylcaprolactam oder deren Gemischen,

c) 1 bis 25 Gew.-% N-Vinylimidazol, 1-Vinyl-2-methyl-imidazol oder deren Gemischen und

d) 1 bis 25 Gew.-% (Meth)acrylsäure erhalten werden und einen K-Wert nach Fikentscher von 15 bis 75, gemessen in Ethanol bei 25 °C, aufweisen.

2. Haarbehandlungsmittel, enthaltend als Filmbildner Copolymerisate gemäss Anspruch 1.

3. Verwendung von Copolymerisaten nach Anspruch 1 als Filmbildner in Haarbehandlungsmitteln.

**Revendications**

1. Copolymères produits par une copolymérisation radicalaire d'un ester alkylique de l'acide (méth)acrylique, d'un monomère azoté hydrosoluble à réaction neutre, d'un monomère à groupes cationiques et d'un acide carboxylique en $C_3$ ou $C_4$ à insaturation oléfinique, caractérisés en ce qu'ils sont préparés par une copolymérisation radicalaire de

a) 20 à 75% en poids de (méth)acrylate d'éthyle, de n-butyle, de butyle tertiaire ou de lauryle ou d'un mélange de ceux-ci,

b) 5 à 50% en poids de N-vinyl-pyrrolidone ou de N-vinyl-caprolactam ou d'un mélange de celles-ci,

c) 1 à 25% en poids de N-vinyl-imidazole ou de 1-vinyl-2-methyl-imidazole ou d'un mélange de ceux-ci et

d) 1 à 25% en poids d'acide (méth)acrylique et possèdent une valeur K selon Fikentscher, déterminée à 25 °C dans l'alcool éthylique, comprise entre 15 et 75.

2. Composition de traitement capillaire, contenant comme substance filmogène un copolymère suivant la revendication 1.

3. Utilisation de copolymères suivant la revendication 1 comme substances filmogènes dans des compositions de traitement capillaire.

**Claims**

1. A copolymer obtained by free-radical copolymerization of an alkyl acrylate or methacrylate, a neutral nitrogen-containing water-soluble monomer, a monomer containing cationic groups, and an olefinically unsaturated carboxylic acid of 3 or 4 carbon atoms, characterized in that it is obtained by free-radical copolymerization of

a) 20 to 75% by weight of ethyl, n-butyl, tert.-butyl or lauryl acrylate or methacrylate or mixtures thereof,

b) 5 to 50% by weight of N-vinylpyrrolidone, N-vinylcaprolactam or mixtures thereof,

c) 1 to 25% by weight of N-vinylimidazole, 1-vinyl-2-methylimidazole or mixtures thereof, and

d) 1 to 25% by weight of acrylic acid or methacrylic acid, and has a Fikentscher K value of from 15 to 75, measured in ethanol at 25 °C.

2. A hair treatment agent which contains, as film former, a copolymer as claimed in claim 1.

3. The use of a copolymer as claimed in claim 1 as film former in hair treatment agents.